# EUROPÄISCHE PATENTANMELDUNG

(11) **EP 1 605 057 A2**
(43) Veröffentlichungstag der Anmeldung: **14.12.2005**
(21) Anmeldenummer: 05012453.6
(22) Anmeldetag: 09.06.2005
(51) Int. Cl.: C12P 21/02

(54) **Verfahren zur Herstellung hyperglykosylierter Proteine**

(30) Priorität: 09.06.2004 DE 102004028208
(71) Anmelder: Heyne, Klaus, 24146 Kiel (DE); Weidinger, Sebastian, 80797 München (DE)
(72) Erfinder: Heyne, Klaus, 24146 Kiel (DE); Weidinger, Sebastian, 80797 München (DE)
(74) Vertreter: Blodig, Wolfgang

(57) **Zusammenfassung**

Die Erfindung beschreibt ein Verfahren zur Herstellung eines hyperglykosylierten Proteins umfassend das Behandeln eukaryotischer Zellen, die das Protein exprimieren, mit Pseudomonas.

## Beschreibung

Die Erfindung betrifft ein Verfahren zur Herstellung von hyperglykosylierten Proteinen in eukaryotischen Zellen. Die Erfindung betrifft ferner die so hergestellten Proteine. Die Erfindung betrifft ferner die Verwendung von Pseudomonas-Toxinen zur Expression hyperglykolysierter Proteine in eukaryotischen Zellen.

Tierische Zellen in Kultur werden industriell als Produktionssysteme für Glykoproteine/konjugate mit korrekten posttranslationalen Modifikationen wie Glykosylierung verwendet. Solche Glykoproteine werden zu vielfältigen diagnostischen und therapeutischen Zwecken verwendet. Die Nützlichkeit von rekombinant in pflanzlichen und tierischen Zellsystemen oder Organismen (z.B. transgene Tiere) hergestellten Proteinen wird jedoch durch spezies-, zell- und organspezifische Glykosylierungs-Vorgängen beschränkt.

Die Glykane, komplexe Kohlenhydratstrukturen, beeinflussen verschiedene Eigenschaften der jeweiligen Trägerproteine im resultierenden Hybridmolekül (Helenius A, Aebi M (2001) Intracellular functions of N-linked glycans. Science 291: 2364-2369; Andersen DC, Krummen L (2002) Recombinant protein expression for therapeutic applications. Curr Opin Biotech 13: 117-123). Das Engineering der Glycosylierung der Glykoproteine aus z.B. Zellkulturen (Gödia F, Cairó JJ (2002) Metabolic engineering of animal cells. Bioprocess Biosyst Engineering 24: 289-298) kann sowohl durch Gentransfer für Glykosylierungsenzyme als auch durch deren Blockierung z.B. mittels "antisense technology" erfolgen. Weiterhin kommen chemische Synthese, enzymatische Transformation und chemoselektive Ligation bei der Gewinnung von Glykoproteinen zum Einsatz (Grogan MJ, Pratt MR, Marcaurelle LA, Bertozzi CR (2002) Homogeneous glycopeptides and glycoproteins for biological Investigation. Ann Rev Biochem 71: 593-634).

Die Glykosylierung von Proteinen ist eine posttranslationale Modifikation. Durch Glykosylierung erfolgt punktgenaue Konditionierung ("Tuning") eines translatierten Proteins für spezielle und wechselnde technische Anforderungen an dessen Funktion. So liegen Glykoproteine generell als heterogene Populationen von verschieden glykosylierten Varianten eines Proteins vor.

Verstärkte Glykosylierung ("hyper-/overglycosylation") bedeutet in der Regel auch verstärkte Ausstattung mit endständig an Glykanstrukturen exponierten Sialinsäuren ("oversialylation"). Sialinsäuren, relativ große, hydrophile und saure Moleküle beeinflussen die biologische Wirkung des jeweiligen Glykoproteins durch ihre Ladung, durch Veränderung von Viskosität und proteolytischer Resistenz, durch Molekülmaskierung (anti-recognition effect) mit veränderter Antigenität und durch Rezeptorbindung (Schauer R (1982) Adv Carbohydrate Chem Biochem 40: 131-234; Lisowska E (2002) CMLS, Cell Mol Life Sci 59: 445-455).

Zum Beispiel kann das Fehlen eines N-Glykans zum Verlust der Enzymaktivität führen (Krankheitsbild Ceroidlipofuszinose: Tsiakas K et al. (2004) Mutation der N-Glykosylierungsstelle N 286 des humanen CLN2 - Proteins führt zum Verlust der enzymatischen Aktivität. APS Fulda (Abstrakt)). Andererseits kann die Verknüpfung mit einem N-Glykan zu erhöhter biologischer Aktivität in vitro bei Calcitonin, einem Peptidhormon, führen (zit. Grogan MJ et al. (2002) Ann Rev Biochem 71: 593-634). Ferner kann ein einzelnes N-Glykan die hormonelle Funktion des von einem Einzel-Gen codierten equinen Peptidhormons Chorion-Gonadotropin zu der des Lutropin wandeln (zit. Grogan MJ et al. (2002)).

Es ist bekannt, dass die posttranslationale Modifikation von Proteinen mit (komplexen) Kohlenhydratstrukturen (Glykanen) die Funktion des Proteins verändern kann. Hochglykosylierte Isoformen von Glykoproteinen sind z.B. wegen der verlängerten Serum-Halbwertszeit im Organismus von therapeutischem Interesse, z.B. wenn das spezielle Glykoprotein z.B. als Zellwachstumsfaktor (Beispiel: Erythropoetin, EPO) oder als Hormon wirksam ist.

"Metabolic engineering of glycosylation" kann durch Gentransfer für Glyko-sylierungsenzyme wie auch durch deren Blockierung, durch genetische Modifikation von Glykosylierungspositionen am Polypeptid (Beispiel: Einführung von Asparaginen in EPO) oder durch Optimierung des Substratangebots für glykosylierende Zellkulturen (WO 99/28455) erreicht werden. Chemische Synthese, enzymatische Transformation und chemoselektive Ligation sind weitere (aufwändigere) Verfahren.

### Beschreibung der Erfindung

All diese Verfahren haben jedoch gewisse Nachteile, die ihre Anwendbarkeit beschränken. Es besteht daher ein Bedarf an weiteren Verfahren, die es gestatten, auf einfache und generell anwendbare Weise den Glykangehalt von Glykoproteinen zu erhöhen.

Diese Aufgabe wird durch das Verfahren von Anspruch 1 gelöst. Die Erfindung stellt ferner nach dem Verfahren der Erfindung erhaltene Proteine zur Verfügung.

Die Erfinder haben überraschenderweise gefunden, dass eine Pseudomonas-Infektion von eukaryotischen Zellen dazu führt, dass die eukaryotischen Zellen Proteine mit einem durchschnittlich höheren Glykangehalt als in Abwesenheit der Infektion produzieren. Eine solche verstärkte Glykosylierung wird hier als Hyperglykosylierung bezeichnet. Die erfindungsgemäße Hyperglykosylierung kann zu einer größeren Anzahl von Glykanen pro Proteinmolekül führen und/oder zu einer Erhöhung der Molekülmasse von an das Protein gebundenen Glykanen. Die Hyperglykosylierung betrifft O-verknüpfte und N-verknüpfte Glykane, vorzugsweise jedoch N-verknüpfte Glykane. Die erfindungsgemäße Hyperglykosylierung lässt sich anhand einer Erhöhung des Molekulargewichts des Proteins im Vergleich zu dem in Abwesenheit der Pseudomonas-Infektion produzierten Protein feststellen. Die Erhöhung des Molekulargewichts kann auch anhand einer zu höheren Molekulargewichten verschobenen Molekulargewichtsverteilung einer Proteinpopulation festgestellt werden.

Das erfindungsgemäße Verfahren eignet sich besonders zur Herstellung pharmazeutischer Proteine. Die Hyperglykosylierung der Proteine führt nach Verabreichung zu einer längeren Verweildauer im Organismus, insbesondere in der Blutbahn, so dass geringere Mengen des pharmazeutischen Proteins verabreicht werden müssen oder die Verabreichung in längeren Zeitabständen vorgenommen werden kann. Dies vermindert Nebenwirkungen und verbessert die Patienten-Compliance.

In dem erfindungsgemäßen Verfahren zur Herstellung eines hyperglykosylierten Proteins wird Pseudomonas dazu verwendet, in eukaryotischen Zellen die Hyperglykosylierung von von den eukaryotischen Zellen exprimierten Proteinen zu induzieren. Der Mechanismus dieser Induktion ist noch nicht geklärt. Es wird jedoch vermutet, dass die Hyperglykosylierung von Pseudomonas-Toxinen, die in die eukaryotischen Zellen eindringen, verursacht wird. Daher stellt die Erfindung die Verwendung von Pseudomonas-Toxinen zur Induktion einer Hyperglykosylierung von Proteinen in eukaryotischen Zellen bereit. Bei dieser Verwendung können auch zellfreie Toxinpräparate oder isolierte Toxine zur Induktion der Hyperglykosylierung eingesetzt werden, wobei die Verwendung des Toxingemischs bevorzugt ist. Der Einsatz der Bakterien in dem erfindungsgemäßen Verfahren hat den Vorteil, dass die Bakterien über ein System zum Einführen der Toxine in die eukaryotischen Zellen verfügen.

Die eukaryotischen Zellen zur Verwendung in dieser Erfindung können pflanzliche Zellen, tierische Zellen oder Insektenzellen sein. Die eukaryotischen Zellen können zu einer Pflanze oder einem Tier gehören, d. h. das erfindungsgemäße Verfahren kann in einer Pflanze oder in einem Tier durchgeführt werden. Das erfindungsgemäße Verfahren wird vorzugsweise in einem Tier oder mit tierischen Zellen durchgeführt. Besonders bevorzugt wird das erfindungsgemäße Verfahren in Säugetierzellen oder Säugetieren durchgeführt, wobei das Verfahren nicht in Menschen durchgeführt wird.

Die Säugetierzellen zur Verwendung in dieser Erfindung können von jedem Säugetier abgeleitet sein oder zu irgendeinem nichtmenschlichen Säugetier gehören. Beispiele für geeignete Säugetierzellen sind unter anderem solche von Nagern, Wiederkäuern, Primaten usw. Konkrete Beispiele sind Maus, Ratte, Hamster, Rind, Schaf, Ziege, Affe oder auch dem Schwein. Bevorzugte Säugetierzellen zur Anwendung der Erfindung in Zellkultur sind Zellen von Primaten, insbesondere menschliche Zellen.

Als Pseudomonaden kann in dieser Erfindung jede Pseudomonasart verwendet werden, die die verwendeten eukaryotischen Zellen infizieren können. Beispiele für Pseudomonasarten sind die unter www.dsmz.de/species/gn302742.htm der Deutschen Sammlung von Mikroorganismen und Zellkulturen GmbH (DSMZ) genannten wie P. aeruginosa, P. putida, P. syringae, P. stutzeri und andere, wobei P. aeruginosa bevorzugt ist. Ferner können ähnliche Bakteriengenera eingesetzt werden wie z.B. Xanthomonaden. Ferner kommen Bakterien aus anderen Genera der Familie Pseudomonadaceae in Frage. Diese Bakterien sind von gängigen Hinterlegungsstellen für Mikroorganismen wie z.B. der DSMZ erhältlich. Geeignete Medien und Wachstumsbedingungen für solche Bakterien sind im Stand der Technik bekannt oder können von der DSMZ zur Verfügung gestellt.

Das erfindungsgemäße Verfahren kann in Zellkultur mit eukaryotischen Zellen durchgeführt werden. Für die Zellkultur sind pflanzliche, tierische und Insektenzellen geeignet. Bevorzugt sind jedoch tierische, insbesondere menschliche Zellen. Die Zellkultur kann im kleinen Maßstab wie z.B. in Zellkulturschalen oder -platten oder in Bioreaktoren durchgeführt werden. Die eukaryotischen Zellen werden dabei vorzugsweise in Suspension gehalten. Verfahren für die Zellkultur sind hinsichtlich Kulturmedien, Temperatur und sonstigen Bedingungen für viele Zelltypen im Stand der Technik bekannt. Die eukaryotischen Zellen werden üblicherweise bis zu einer geeigneten Zelldichte herangezogen. Dann wird das Kulturmedium mit Pseudomonas-Bakterien versetzt. Das Kulturmedium als auch die Kulturbedingungen müssen sowohl für die eukaryotischen Zellen als auch für Pseudomonas geeignet sein, was wegen der Anspruchslosigkeit von Pseudomonas in der Regel unproblematisch ist. Antibiotika, gegen die die eingesetzten Pseudomonaden nicht resistent sind, sollten nicht vorliegen. Hinsichtlich der Menge der zugesetzten Pseudomonaden bestehen keine besonderen Beschränkungen, sofern, eventuell nach entsprechender Vermehrung, eine ausreichende Dichte erreicht werden kann, um die Mehrzahl der eukaryotischen Zellen zu infizieren. Die Zellkultur sollte temperaturkontrolliert sein. Die Temperatur des Mediums sollte zwischen 30 und 42°C, vorzugsweise um 37°C, betragen, da die Infektivität der Pseudomonaden bei dieser Temperatur am größten ist. Nach erfolgter Infektion der eukaryotischen Zellen kann bei Bedarf durch Zusatz von Antibiotika ein zu starkes Wachstum der Pseudomonaden kontrolliert werden.

Beispiele für Säugetierzellen, die für das Verfahren in Zellkultur verwendet werden können, sind CHO-Zellen, 293-Zellen, HeLa-Zellen. Diese und andere Zellen sind kommerziell erhältlich z.B. von Invitrogen oder Gibco. Geeignete Kulturmedien sind von denselben Herstellern erhältlich.

Die Hyperglykosylierung setzt üblicherweise wenige Stunden nach Infektion mit Pseudomonas ein und kann über mehrere Tage bis Wochen anhalten. Die Dauer der Hyperglykosylierung hängt von der Dauer der Infektion ab.

Nach erfolgter Produktion des gewünschten hyperglykosylierten Proteins in Zellkultur kann das Protein isoliert werden. Im Fall eines sekretierten Proteins kann dieses aus dem Kulturmedium isoliert werden, z.B. nach Abtrennen der zellulären Bestandteile. Im Fall von nicht sekretierten Proteinen können diese aus den eukaryotischen Zellen isoliert werden. Bei der Isolierung und Reinigung des hyperglykosylierten Proteins kommen bekannte Verfahren der Proteinreinigung zur Anwendung, die sich vor allem nach dem zu isolierenden Protein richten.

Die Produktion des hyperglykosylierten Proteins oder der hyperglykosylierten Proteine kann durch Probenentnahme aus der Zellkultur und geeignete Analyse der Proteine z.B. mittels Gelelektrophorese, Isoelektrischer Fokussierung (IEF), Gelpermeationschromatographie oder Massenspektroskopie verfolgt werden. Auf diese Weise kann der optimale Zeitpunkt zur Ernte bestimmt werden. Wird das gewünschte Protein in das Kulturmedium sekretiert, kann dieses auch kontinuierlich entnommen werden, um daraus das gewünschte hyperglykosylierte Protein zu isolieren.

Bei der Herstellung des hyperglykosylierten Proteins in einem Tier muss das Tier mit Pseudomonas behandelt werden. Vorzugsweise muss das Tier so mit Pseudomonas behandelt werden, dass eine Infektion der Zellen stattfinden kann, die das Protein exprimieren. Vorzugsweise wird das Säugetier mittels Infusion in ein Blutgefäß oder Lymphgefäß mit Pseudomonas behandelt. Findet die Expression des hyperglykosylierten Proteins in einem bestimmten Organ oder Gewebe des Tieres statt, kann das betreffende Organ oder Gewebe durch Infusion in ein das betreffende Organ oder Gewebe versorgendes Blutgefäß mit Pseudomonas infiziert werden. Findet die Expression z.B. in der Leber statt, so kann Pseudomonas durch einen Port in die Pfortader eingeleitet werden. Das Infusionsmedium sollte das Überleben der Pseudomonaden unterstützen. Hierfür kommt eine isotone Kochsalzlösung mit einer Kohlenstoffquelle wie einem Kohlenhydrat in Frage. Die Konzentration der Bakterien im Infusionsmedium hängt von der Dauer der Infusion ab. Vorzugsweise wird eine Dauerinfusion über mehrere Tage durchgeführt. In diesem Fall genügt eine geringe Konzentration der Pseudomonaden von beispielsweise einer optischen Dichte bei 600 nm (OD600) von 0,01 bis 0,1. Nach erfolgter Expression des hyperglykosylierten Proteins kann dieses aus dem Tier isoliert werden.

Vorzugsweise werden nach dem erfindungsgemäßen Verfahren sekretorische Proteine hergestellt, die das Tier in die Blutbahn abgibt. In diesem Fall kann das hyperglykosylierte Protein aus dem Blutplasma des Säugetiers gewonnen werden. Eine weitere vorteilhafte Methode besteht darin, das gewünschte Protein in die Milch von beispielsweise Kuh, Ziege oder Schaf abzugeben, aus der es dann isoliert werden kann. Für Immunproteine wie Antikörper eignet sich die Gewinnung der Lymphe eines Tiers, aus der die Immunproteine gewonnen werden können.

Das erfindungsgemäße Verfahren eignet sich zur Herstellung von Proteinen, die natürlicherweise in den verwendeten eukaryotischen Zellen exprimiert werden oder zur Herstellung von heterologen Proteinen. Da das erfindungsgemäße Verfahren zu einer Hyperglykosylierung vieler der in einer infizierten Zelle produzierten Proteine führt, kann eine Vielzahl hyperglykosylierter Proteine gleichzeitig erhalten werden kann. Um ein gewünschtes Protein in hoher Ausbeute zu erhalten, sollte das gewünschte Protein mit den Methoden des Genetic Engineering überexprimiert werden.

Soll ein heterologes Protein hergestellt werden, wird eine für das gewünschte Protein kodierende Nukleinsäure mit geeigneten Regulationselementen für die Expression mit einem Vektor in die eukaryotischen Zellen eingebracht. Auch im Fall der Produktion eines zelleigenen Proteins kann durch genetische Modifikation eine Überexpression des gewünschten Proteins erreicht werden, wodurch die Menge des gewünschten Proteins relativ zum normalen Proteinkomplement der Zellen erhöht werden kann. Vorzugsweise werden bei dem erfindungsgemäßen Verfahren daher die eukaryotischen Zellen mit einer exprimierbaren DNA transfiziert, die für das Protein kodiert. Die eukaryotischen Zellen können transient oder stabil mit einer Nukleinsäure transfiziert werden. Verfahren zur Transfektion der für das erfindungsgemäße Verfahren in Frage kommenden eukaryotischen Zellen sind im Stand der Technik bekannt. Für die Transfektion von Pflanzenzellen eignen sich besonders Vektorsysteme, die auf pflanzlichen RNA-Viren wie dem Tabakmosaikvirus beruhen. Für die Transfektion von Insektenzellen ist das Baculovirus-System gut etabliert. Für die Transfektion von tierischen Zellen können ebenfalls virale Systeme angewandt werden, z.B. solche, die auf Adenoviren, auf Adeno-assoziierten Viren oder auf Retroviren beruhen. Virale Transfektionssysteme eignen sich besonders für die transiente Transfektion, insbesondere für Zellkultursysteme im großen Maßstab. Virale Expressionssysteme zeichnen sich häufig durch die Produktion großer Mengen des gewünschte Proteins aus. Bei einer transienten Transfektion z.B. mit einem viralen Expressionsystem kann die Verabreichung der viralen Vektoren und der Pseudomonaden gleichzeitig oder nacheinander erfolgen.

Selbstverständlich kann die Erfindung auch mit stabil transfizierten eukaryotischen Zellen in Zellkultur oder mit transgenen Tieren durchgeführt werden. Im Fall von transgenen Tieren kann das gewünschte Protein z.B. mit Hilfe von gewebsspezifischen Promotoren in einem gewünschten Gewebe durchgeführt werden, so dass das gewünschte Protein in ein gewünschtes Extrazellulärkompartiment wie den Blutkreislauf oder die Milch von weiblichen Tieren (Kühe, Schafe, Ziegen) sekretiert wird und aus diesen Medien gewonnen werden kann. Ein Beispiel hierfür ist die Expression von Lysostaphin in der Milch von Kühen (Nature Biotechnology 23 (2005) 445).

Um die Sekretion des gewünschten Proteins aus den exprimierenden Zellen zu ermöglichen, kann der für das gewünschte Protein kodierenden Nukleinsäuresequenz eine für ein Sekretionssignalpeptid kodierende Sequenz vorangestellt werden.

Bei Bedarf kann in eine für ein gewünschtes Protein kodierende Nukleinsäure eine oder mehrere Mutationen eingeführt werden, z.B. um Erkennungssequenzen für eine N-Glykosylierung zu schaffen oder auch abzuschaffen. Insbesondere kann eine (oder mehrere) Erkennungssequenz Asn-X-Thr/Ser für eine N-Glykosylierung in ein gewünschtes Protein eingeführt werden, um die Glykosylierung des Proteins zu erhöhen.

Das erfindungsgemäße Verfahren ist allgemein anwendbar. Proteine, die hyperglykosyliert werden können, sind pharmazeutische Proteine, deren Halbwertszeit im Körper nach Verabreichung an einen Patienten erhöht werden soll. Pharmazeutische Proteine können zu den folgenden Gruppen gehören: Blutgerinnungsfaktoren, Polypeptidhormone, Immunproteine, Cytokine, Interferone, Wachstumsfaktoren, Proteaseinhibitoren. Beispiele sind Proteine der Immunantwort (monoklonale Antikörper, Einzelstrangantikörper, T-Zellrezeptoren usw.), Colony-Stimulating-Factors, Relaxine, Polypeptidhormone wie Somatotropin (HGH) und Proinsulin, Cytokine und deren Rezeptoren, Interferone, lysosomale Enzyme, fibrinolytische Polypeptide, Trypsin, Trypsinogen, α1-Antitrypsin (AAT) und andere Serpine, Serumalbumin, Glucocerebrosidasen, natives Cholera-Toxin B, Thrombin, Granulocyten-Macrophagen-Colony-Stimulating-Factor (GM-CMF), Lactoferrin, Oleosin, Prothrombin, alpha-Galactosidase usw.

Ferner ermöglicht die Erfindung die Hyperglykosylierung von Glykolipiden.

### Kurze Beschreibung der Figuren

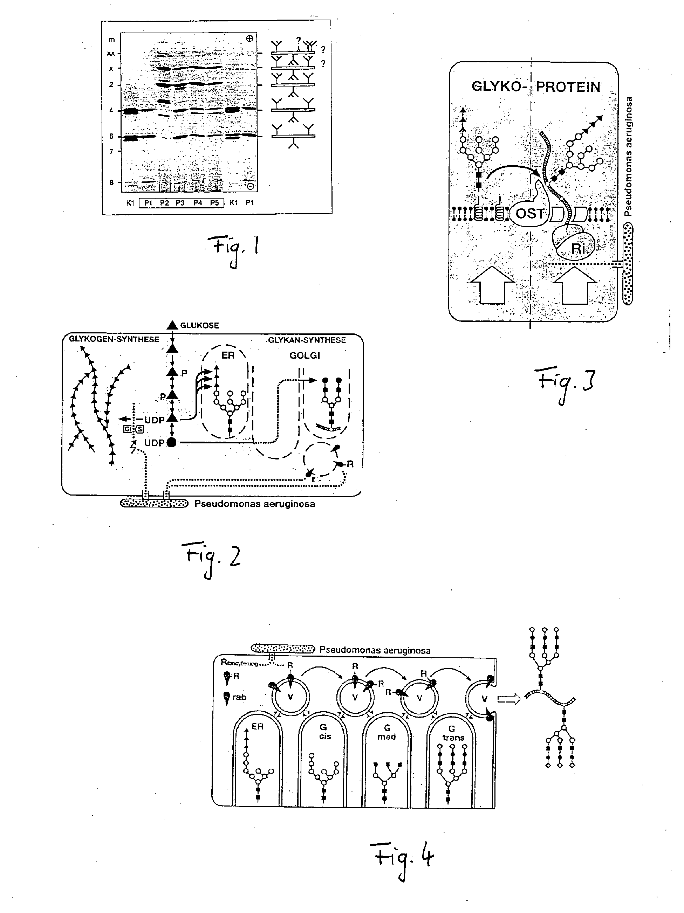

Fig. 1: Pseudomonas aeruginosa-induzierte (abnorme) Hyperglykosylierung/-sialylierung von humanem α1-Antitrypsin (AAT). Isoelektrische Fokussierung von Serumproben mit Coomassie-Färbung (links) sowie schematische Molekülstrukturen (rechts).

Das IEF zeigt Fraktionen m, x und xx (Ordinate) im Zeitraum P2 bis P5 (Abszisse).m: alpha-1-Phänotyp-Fraktionen (Standardnomenklatur der Proteinmuster). x und xx waren bisher unbenannt. Spuren K1 und P1-P5 zeigen Phänotyp-Muster (K1 = Kontrolle, Phänotyp M 1 M2); P bezeichnet Proben aus einem Patienten mit P. aeruginosa-Sepsis (siehe Beispiel). P1: normaler Phänotyp M1M1 des Patienten; P2: Phänotyp an Tag 6 der Infektionskrankheit bei entfernter Infektionsursache; P3: Phänotyp an Tag 12 der postinfektiösen Krankheit; P4: Phänotyp an Tag 38, P5: Phänotyp an Tag 48 des postinfektiösen Zustandes. Für experimentelle Details siehe WEIDINGER et al. (1982) Z. Rechtsmed. 88,203-211; WEIDINGER, CLEVE (1984) Electrophoresis 5, 223-226.

Die schematische Molekülstrukturen rechts neben den IEF-Banden zeigen Glykaneinheiten auf dem Proteinrückgrat (lineare Doppellinie). Die Y-förmigen Symbole deuten die Struktur von Glykaneinheiten an. ? bezeichnet postulierte Molekülstrukturen.

Fig. 2: Pseudomonas aeruginosa - induzierte Favorisierung des Metabolit (UDP-Glukose UDP-Δ; UDP-Galaktose UDP-o) - Flusses zur Glykan-Synthese bei behinderter Glykogen-Synthese sowie Beeinflussung (via Ribosylierung (R) von Rab (r)-Proteinen) vesikulären Substrattransportes. Stoffwechselschema.

Fig. 3: Pseudomonas aeruginosa - induzierte Adjustierungsstörung von Polypeptid-synthese und Glykosylierungspotenz. Schematische Darstellung (OST: Oligosaccha-ryltransferase, Ri: Ribosom).

Fig. 4: Pseudomonas aeruginosa - induzierte Änderung der "Perkolation" von Glykokonjugaten in endoplasmatischem Retikulum (ER), Golgi-System (G) sowie Transportvesikel (V) via Ribosylierung (R) von Rab (r)-Proteinen bzw. -Effektoren. Schematische Darstellung

### Detaillierte Beschreibung der Erfindung

Die mit der Erfindung zu erzielenden Vorteile sind die Möglichkeiten, welche ein "biologisch"-mikrobiell induzierter (genereller) Hyperglykosylierungs-Status eines Organismus bietet: Gewinnung hyperglykosylierter Isoformen diverser Glykoproteine, welche bereits das Synthesezellen-eigene "Qualitäts-Kontroll-System" passierten. Diese hyperglykosylierten Isoformen stehen nach Ausschöpfung der zellulären Glykosylierungspotenzen gegebenenfalls für weiteres biochemisches "engineering" zur Verfügung.

Das Beispiel zeigt die Hyperglykosylierung/Hypersialysierung von α1-Antirypsin (AAT) in einem mit P. aeruginosa infizierten Säugling. Die Hyperglykosylierung/Hypersialylierung wurde durch Isoelektrische Fokussierung nachgewiesen (Fig. 1).

Bei der (6-wöchigen) Persistenz hyperglykosylierter/-sialylierter Isoformen des AAT im Serum könnten sowohl Dauer der P. aeruginosa (P.a.)-induzierten hepatischen Glykosylierungsanomalie als auch verlängerte Serum-Halbwertszeit hyperglykosylierter/-sialylierter Isoformen ursächliche Faktoren sein.

Die Deutung des beobachteten Hyperglykosylierungs-Phänomens berücksichtigt die Zunahme von anomalen Fraktionen des AAT, welches der Zunahme an negativ geladenen (terminalen) Sialinsäure-Molekülen ("Hypersialylierung") gleichzusetzen ist. Unsere (spekulative) Struktur-Zuordnung (Fig. 1, rechts) umfasst:
- den Ersatz biantennärer (N-) Glykane durch tri-(tetra-)antennäre,
- die (normabweichend zusätzliche) Glykosylierung einer potentiellen, unter Normverhältnissen unbesetzten (Mills K et al. (2003) Glycobiology 13 (2): 73-85) und wohl unbesetzbaren (Freeze HH, Westphal V (2001) Biochimie 83: 791-799; Marth JD in: Varki A et al. (Eds.) Essentials of Glycobiology. Cold Spring Harbor Laboratory Press, New York, p.88) Glykosylierungsposition (AAT-sequon 391-393: -Asn-Pro-Thr sowie

### - zusätzliche ("molekularpathologische") O-Glykosylierung.

Den langfristigen Nachweis der hyperglykosylierten/-sialylierten Isoformen des AAT in vivo sehen wir als Kriterium für jene Molekülformen an, welche das zelluläre humane Qualitäts-Kontrollsystem zu passieren vermögen.

Das zu schützende Verfahren beruht auf der Analyse eines speziellen para- und postinfektiösen Hyperglykosylierungsphänomens unter Berücksichtigung des aktuellen Kenntnisstandes über
- Einflußfaktoren auf Glykosylierungsvorgänge sowie
- "zytotoxische" Virulenzfaktoren der Bakterien-Spezies Pseudomonas aeruginosa, einer Gruppe aerober opportunistischer Infektionserreger, von der ein spezieller Einfluß auf Glykosylierungsvorgänge in Zellen infizierter Systeme/Organe/Organismen uns bisher nicht bekannt geworden ist.

Pseudomonas aeruginosa verfügt über zwei besondere Wirkungsmechanismen/-Systeme, über welche - von uns postuliert - auch Glykosylierungsvorgänge infizierter Zellen moduliert werden können:
1. ein "type III protein secretion system", wirkend als Injektionsapparat für bakterienspezifische Wirkstoffe ("Toxine"), hier: Pseudomonas aeruginosa - Exo S, Exo T, Exo Y (Galàn JE, Collmer A (1999) Type III secretion machines: bacterial devices for protein delivery into host cells. Science 284: 1322-1328; Hueck CJ (1989) Microbiol Mol Biol Rev 62 (2): 379-433 (s.a. Lit. 13)),
2. ein spezifisches "Quorum sensing"-system (Lit. 36a, 36b), dessen Signalstoffe nicht allein die Prokaryontenfunktionen (Whiteley M et al. (1999) PNAS 96 (24): 13904-13909), sondern - so die Acyl-Homoserin-Lactone - auch in Eukaryonten ("Wirtszellen") über die Induktion proinflammatorischer Zytokine (z.B. IL-8) (Lit. 24) und Chemokine Induktion und Ablauf einer Akute Phase - Reaktion beeinflussen können.

Das spezielle para- und postinfektiöse Hyperglykosylierungs-Phänomen kam zur Beobachtung bei Glykosylierungsanalysen mittels isoelektrischer Fokussierung (Weidinger S et al. (1985) Hum Genet 71: 27-29; Heyne K, Henke-Wolter J (1989) Eur J Pediat 148: 341-343) am "Modell-Glykoprotein" α1-Antitrypsin (AAT), einem Serpin aus vorwiegend hepatischem Syntheseort, von bekannter Struktur (Huber R, Carrell W (1991) in Neurath H (Ed.): Perspectives in Biochemistry Vol. II, Amer Chem Soc, Washington, pp. 53-68) und bekannter Glykosylierung (3 komplexe, bi- bzw. triantennäre N-Glykane).

### Pathophysiologische und biochemische Aspekte der erfindungsgemäßen Hyperglykosylierung

### A. Para- / postinfektiöse Induktion von Glykosylierungsenzymen

### 1. Unspezifische Akute Phase-Reaktion

Das biologische Phänomen der Akute Phase - Reaktion bzw. des "systemic inflammatory response syndrome " (SIRS) eines Organismus (Kushner I (1982) Ann NY Acad Sci 389: 39-48) nach Verletzung (Zellschädigung) und / oder Infektion führt zur Verschiebung des Glykoprotein-Mikroheterogenitätsmusters zu höher glykosylierten Isoformen . Am "Modell"-Glykoprotein α1-Antitrypsin (Vaughan L, Carrell RW (1981) Biochemistry 2: 461-467; Heyne K et al. (1988)) ließ sich die verstärkte (hepatische) Synthese hochglykosylierter /-sialylierter Isoformen in der para- bzw. postinfektiösen entzündlichen Akute Phase - Reaktion am Verteilungsmuster von Isoformen mit biantennären und triantennären N-Glykanen differenzieren (Riley RS et al. (1990) Clin Immunol 10 (7): 95-99; Heyne K, Weidinger S (1990) Infection 18: 394-395).

Diese Hyperglykosylierung/-sialylierung in der Akute Phase-Reaktion erscheint als Folge unspezifisch erhöhter Aktivität von Glykosyltransferasen (Lombart C et al. (1980); Kaplan HA et al. (1983); Fraser IH et al. (1984) Biochim Biophys Acta 799: 102-105; Olson FJ et al. (2002)), die Glykosyl-(Sialyl-) transferase selbst "appears to behave like an acute phase reactant" (16, 18).

### 2. P.a. - assoziierte Mechanismen

Pseudomonas aeruginosa - spezifische Akute Phase - Reaktion - Induktion (s.o.) via "type III protein secretion system" sowie "quorum sensing system": Pseudomonas aeruginosa aktiviert den Transkriptionsfaktor NF-kappa B (7, 19, 23, 35) sowie Interleukin-8 (24). Im Epithelzell-Modell "secreted factors from P. aeruginosa shift the inflammatory / anti-inflammatory balance to favor a prolonged or excessive pro-inflammatory state" (7).

### B. P.a. - induzierte Favorisierung des anabolen Substratflusses der Glykansynthese

UDP-Glukose, der energiereiche Glukose-Metabolit, ist Substrat anaboler Stoffwechselwege - sowohl für das Polysaccharid Glykogen (katalysiert von Glykogen-Synthase GS (Fig. 2)) als auch für die Oligosaccharide der (N-) Glykane (z.B. via Glukosylierung der Vorläufer-Glykane ("lipid-linked oligosaccharide precursor", LLO) zur Translokation vom Dolichol zum Polypeptid bzw. als Substrat der 4-Epimerase-Reaktion zur UDP-Galaktose (Fig. 2). Siehe Referenzen (3) und (9). Gemäß (9) stellen LLO-Komponenten limitierende Schritte für die N-Glykosylierung dar.

### Pseudomonas aeruginosa induziert Proteinkinase C (14)

Proteinkinase C (PKC) der Leberzelle inaktiviert durch Phosphorylierung die Glykogen-Synthase (21). Blockade der Glykogensynthase favorisiert den UDP-Glukose / UDP-Galaktose - Substratfluss zur (N-) Glykan-Synthese (siehe Fig. 2).

Mangelnde Verfügbarkeit von UDP-Glukose bzw. UDP-Galaktose bei genetischem Transferase-Defekt ist Ursache von Congenital disorders of glycosylation (CDG), der Typen CDG Ic bzw. CDG IId beim Menschen (11, 34).

Pseudomonas aeruginosa - Sepsis beim Menschen wird klinisch begleitet von niedrigen Serum-Glukose-Konzentrationen (39a) als möglicher Folge geringer Glykogen-Depots.

### C. Glykosylierungsfördernde Synthese-Adjustierungsstörung bei P.a. - induzierter Behinderung anteiliger Polypeptidsynthese

Die Synthese von Glykoproteinen erfordert gleichsinnige, adjustierte Synthese der Vorläufermoleküle (N-) Glykan und Polypeptid; ihre (posttranslationale) Verknüpfung erfolgt durch die Oligosaccharyltransferase (OST) (siehe Fig. 3) ("...,coupling the rate of protein synthesis with that of LLO synthesis,..."(9)).

Pseudomonas aeruginosa - Exotoxin A hemmt die Polypeptidsynthese durch Inaktivierung (ADP-Ribosylierung) des ribosomalen "elongation factor 2" (15).

Isolierte Blockade der Polypeptidsynthese - via Cycloheximid-Hemmung ribosomaler Peptidyltransferase-Aktivität (38, 39) - stört die Adjustierung und stimuliert die Glykosylierung vorhandener Akzeptormoleküle (39) bzw. Akzeptorsequons (38); Cycloheximid sowie Anisomycin bedingte Proteinsynthese-Hemmung vermochte die mRNA-Expression einer Glykosyl- (Galaktosyl-)Transferase zu induzieren, begleitet von erhöhtem Niveau an Enzymprotein und -aktivität (22a).

### D. P.a. - induzierte Veränderung des intrazellulären Transports werdender

Glykoproteine mit konsekutiv verändertem (verlängertem) Enzymkontakt sowie optimierter Nutzung von potentiellen Glykan-Akzeptorsequons

Siehe "Other factors affecting N-glycan diversification include sugar nucleotide metabolism, transport rates in the ER and Golgi,..." (Marth JD in: Varki, A et al. (Eds.) (1999). Essentials in Glycobiology. Cold Spring Harbor Laboratory Press. Cold Spring Harbor, New York). Neu synthetisierte Proteine, die länger im ER sind, können hyperglykosyliert werden, indem Glycanketten an Sequons gebunden werden, die unter normalen Umständen frei bleiben (Freeze HH, Westphal V (2001)).

Den intrazellulären Transport (mit retrograden Komponenten) der werdenden Glykokonjugate besorgen Vesikel (V in Fig. 2 u. 4) in einem mit "Perkolation" vergleichbaren und bezeichneten Prozess (27, 30). Am energiebedürftigen und zielgerichteten Transportvorgang sind "switch"-Proteine mit GTPase-Aktivität (rab-Proteine; Fig. 4) beteiligt (4, 26, 28, 36). Die Art des rab-Proteins bestimmt die Spezifität des kompartimentellen Transports, die Kinetik der rab-GTPase-Aktivität bestimmt dessen Dynamik.

rab-Proteine (speziell rab 5 (32, 41)) können als Zeitgeber (timer) des vesikulären Transports und somit des Aufenthaltes der werdenden Glykokonjugate in Glykosylierungskompartimenten (endoplasmatisches Retikulum (ER), Golgi-System (G), Transportvesikel bzw. Endosom (V) (in Fig. 4)) wirken (41).

Mutiertes rab 5 von reduzierter GTPase-Aktivität führte zu verstärkter Internalisierung und behindertem "recycling" des Glykoproteins Transferrin (37). Mutiertes rab 11 von reduzierter GTP-ase-Aktivität führte zu einer erhöhter Rate eines Transferrinefflux, mutiertes rab 11 von defizienter GTP-Bindung jedoch zu vermindertem (exozytotischen) Transferrinefflux (6). Außerdem können GTPase-aktivierende Proteine (GAP) die Wirkung / Wirkungsdauer der "switch"-Proteine inaktivieren / verkürzen durch Auslösen der Hydrolyse von gebundenem GTP zu GDP (36). Pseudomonas aeruginosa Exo T - GAP behindert die " Internalisierung der Mikrobe in Makrophagen durch Wirkung auf das "actin cytosceleton" (20a).

ADP-Ribosylierung von "switch"-Protein bewirkt ebenfalls und stets deren Funktionsänderung (1, 5, 10, 40). Überdies vermag die Überexpression von rab-Protein (rab 7) den vesikulären Transport zu verlangsamen: "Late endocytic vesicles loaded with cholesterol lose their dynamic properties, and became essentially immobile, ...-..., wild-type rab 7 overexpression mimics the effect of cholesterol on motility in control cells" (20).

Vesikulärer Transport bzw. "Perkolation" könnten folglich verlangsamt und damit effektiver werden sowohl durch eine Funktionsänderung von rab-Proteinen als auch durch Überexpression oder Überinduktion von speziellem rab-Protein. Im Wirkungsprofil von Pseudomonas aeruginosa sind drei Mechanismen möglich:
- Pseudomonas aeruginosa - Exoenzym S (Exo S) ADP-ribosyliert diverse GTP-bindende Proteine (1, 5, 8, 31). Als endogene in vivo-Substrate der (Exo S-) ADP-Ribosyl-Transferase-Aktivität wurden u.a. identifiziert (3): rab 5, rab 7, rab 8, und rab 11.

Entsprechend bekannter Zuordnung der rab-Proteine zu Kompartimenten des vesikulären Transports (26) könnte Pseudomonas aeruginosa folgende Transportwege/-abläufe beeinflussen:
rab 5: Plasma membrane → early endosome transport and homotypic fusion between early endosomes
rab 7: Transport from early to late endosomes and lysosomes
rab 8: Golgi → plasma membrane transport
rab 11: (Coat component of COP II vesicles); transport through recycling endosomes
   - Pseudomonas aeruginosa - Exotoxin T (Exo T) (17) sowie Exo S (10, 31) wirken als GTPase-aktivierende Proteine (GAP) (20a) für diverse GTP-bindende "switch"-Proteine (17).
   - Pseudomonas aeruginosa induziert (14) das "ras-related protein rab 25" (endosomeassociated (40)) sowie das "GTP-binding protein rho B".

In einem temperaturabhängigen Vorgang (zur effektiven Expression des "type III protein secretion system"(13), über welches Pseudomonas aeruginosa Exo S und Exo T in die eukaryote Zelle gelangen) bewirkt Pseudomonas aeruginosa vermutlich die Glykosylierungs-begünstigende Veränderung der intrazellulären Dynamik.

Etwa 2/3 der potentiellen N-Glykan-Akzeptor-Sequons -Asn-X-Ser/Thr- können bei gut charakterisierten Glykoproteinen glykosyliert sein (2). Bei der Zugänglichkeit dieser Sequons für N-Glykane spielt die dem Sequon jeweils folgende Aminosäure Y eine Rolle. Pro wie auch Trp in Y-Position des erweiterten Akzeptor-Sequons -Asn-X-Ser/Thr-Y- behindern die Glykosylierung ähnlich wie bei ihrer Stellung in X-Position (25). In einem Sequon -Asn-Gly-Thr-Pro- war der Glykosylierungsgrad dieses behindert zugänglichen Akzeptor-Sequons "correlated inversely with the rate of translation" (38). Molekulare Analyse der Erkrankung MARFAN-Syndrom ergab, dass normales Profibrillin durch verübergehendes Zurückhalten im ER mittels BFA (brefeldin A) hyper-N-glycosyliert werden kann (28).

### Beispiel

Die Pseudomonas aeruginosa (P.a.)-induzierte Hyperglykosylierung/-sialylierung des "Modell"-Glykoproteins Alpha-1-Antitrypsin (Sepsis beim Säugling; Anlage Fig. 1) kann die Wirkung des erfindungsgemäßen Verfahrens illustrieren. Die hyperglykosylierten/-sialylierten Isoformen (speziell Fraktionen m x und m xx n den Proben P2 bis P5) sind im Zeitraum von bis zu 6 Wochen nachweisbar.

Die Wirkung der P. aeruginosa Infektion ist in Fig. 1 gezeigt. Die Wirkungsweise ist schematisch in Fig. 2-4 dargestellt.

Beobachtung: Ein 4 Wochen alter menschlicher Säugling (A, Z) erkrankte postoperativ (Operation: Ventrikulo-atrialer Shunt zur Hydrozephalus-Therapie) an "hämatogener" systemisch-septischer Infektion. Heilung von der Infektion erfolgte nach Antibiose (seit 1. postoperativem Tag) und Entfernung des infizierten Drainage (Shunt-)-Systems. Nachweis von Pseudomonas aeruginosa als Infektionserreger.

Ein abnorm ausgeprägtes Hyperglykosylierungsmuster war mittels isoelektrischer Fokussierung nachweisbar von Tag 6 (Erstanalyse) (P2, Fig. 1) bis (mindestens) Tag 48 (P5, Fig. 1) nach Infektionsbeginn. Das normale genetische (Glykoprotein-) Muster M1M1 war bei Analyse am Tag 91 post infectionem (P1, Fig. 1) erkennbar.

Epikritisch entsprach die Hyperglykosylierungsphase des humanen (hepatischen) Glykoproteins von mindestens 42 Tagen Dauer dem Folgezustand einer iatrogenen systemischen Pseudomonas aeruginosa - Infektion durch intravenöse Infusion der Infektionserreger aus infiziertem Liquor cerebrospinalis-Drainage-System.

### Literatur

1. Aktories K (1997) Bacterial toxins that target Rho proteins. J Clin Invest 99: 827-829
2. Apweiler R, Hermjakob H, Sharon N (1999) On the frequency of protein glycosylation, as deduced from analysis of the SWISS-PROT database. Biochim Biophys Acta 1473: 4-8
3. Brockhausen I, Schutzbach J, Kuhns W (1998) Glycoproteins and their relationship to human disease. Acta Anat 161: 36-78
4. Chavrier P, Goud B (1999) The role of ARF and Rab GTPases in membrane transport. Curr Opin Cell Biol 11: 466-475
5. Coburn J, Wyatt RT, Iglewski BH, Gill DM (1989) Several GTP-binding proteins, including p21^{c-H-ras}, are preferred substrates of pseudomonas aeruginosa exoenzyme S. J Biol Chem 264: 9004-9008
6. Cox D, Lee DJ, Dale BM, Calafat J, Greenberg S (2000) A Rab 11-containing rapidly recycling compartment in macrophage that promotes phagocytosis. PNAS 97: 680-685
7. Denning GM, Munson KL (2002) Secretory factors from pseudomonas aeruginosa alter levels of inflammatory mediators expressed by human airway epithelial cells: Upsetting the inflammatory balance. FASEB J 16 (4): A 303 Abstr. 239.3
8. Donnenberg MS (2000) Pathogenic strategies of enteric bacteria. Nature 406: 768-774
9. Freeze H, Westphal V (2001) Balancing N-linked glycosylation to avoid disease. Biochimie 83: 791-799
10. Goehring U-M, Schmidt G, Pederson KJ, Aktories K, Barbieri JT (1999) The N-terminal domain of pseudomonas exoenzyme S is a GTPase-activating protein for Rho GTPases. J Biol Chem 274: 36369-36372
11. Hanßke B, Thiel C, Lübke T, Hasilik M, Höning S, Peters V et al. (2002) Deficiency of UDP-galactose: N-acetylglucosamine β-1,4-galactosyl-transferase I causes the congenital disorder of glycosylation type II d. J Clin Invest 109: 725-733.
12. Heyne K, Tegtmeyer FK, Henke-Wolter J, Ziesenitz S (1988) α1-Antitrypsin als Modell-Glykoprotein: Modifikation des genetisch determinierten Phänotyps durch nutritivmetabolische Einflüsse und in der Akute-Phase-Reaktion. Mschr Kinderheilk 136: 511 (Abstr.)
12a.Heyne K, Weidinger S (1990) Transient aberrancy of α1-antytrypsin glycoprotein microheterogeneity in pseudomonas aeruginosa septicaemia. Infection 18 (6): 394-395
13. Hueck CJ (1998) Type III protein secretion systems in bacterial pathogens of animals and plants. Microbiol Mol Biol Rev 62: 379-433
14. Ichikawa JK, Norris A, Bangera MG, Geiss GK, van't Wout AB, Bumgarner RE, Lory S (2000) Interaction of pseudomonas aeruginosa with epithelial cells: Identification of differentially regulated genes by expression microarray analysis of human cDNAs. PNAS 97: 9659-9664
15. Iglewski BH, Kabat D (1975) NAD-dependent inhibition of protein synthesis by pseudomonas aeruginosa toxin. PNAS 72: 2284-2288
16. Kaplan HA, Woloski BMRNJ, Hellman M, Jamieson JC (1983) Studies on the effect of inflammation on rat liver and serum sialyltransferase. J Biol Chem 258: 11505-11509
17. Kazmierczak BI, Engel JN (2002) Pseudomonas aeruginosa Exo T acts in vivo as a GTPase-activating protein for RhoA, Rac1, and Cdc42. Infection and Immunity 70: 2198-2205
18. Kleene R, Berger EG (1993) The molecular and cell biology of glycosyltransferases. Biochim Biophys Acta 1154: 283-325
19. Lee DJ, Cox D, Li J, Greenberg S (2000) Rac1 and Cdc42 are required for phagocytosis, but not NF-κB-dependent gene expression, in macrophages challenged with pseudomonas aeruginosa. J Biol Chem 275: 141-146
20. Lebrand C, Corti M, Goodson H, Cosson P, Cavalli V, Mayran N, Faure J, Gruenberg J (2002) Late endosome motility depends on lipids via the small GTPase Rab 7. EMBO Journal 21: 1289-1300
20a.Litvak Y, Selinger Z (2003) Bacterial mimics of eukaryotic GTPase-activating proteins (GAPs). Trends Biochem Sci/TIBS 28 (12): 628-631
21. Lodish H, Berk A, Zipurski SL, Matsudaira P, Baltimore D, Darnell J (2000) Molecular Cell Biology, 4^{th} Edition, W.H. Freeman Co, New York
22. Lombart C, Sturgess J, Schachter H (1980) The effect of turbentine-induced inflammation on rat liver glycosyltransferases and golgi complex ultrastructure. Biochim Biophys Acta 629: 1-12
22a.Masibay AS, Damewood GP, Boeggeman E, Quasba PK (1991) Expression of β-1,4-galactosyltransferase gene during 3Te cell growth. Biochim Biophys Acta 1090: 230-234
23. DiMango E, Ratner AJ, Bryan R, Tabibi S, Prince A (1998) Activation of NF-κB by adherent pseudomonas aeruginosa in normal and cystic fibrosis epithelial cells. J Clin Invest 101: 2598-2605
24. DiMango E, Zar HJ, Bryan R, Prince A (1995) Diverse pseudomonas aeruginosa gene products stimulate respiratory epithelial cells to produce interleukin -8. J Clin Invest 96: 2204-2210
25. Mellquist JL, Kasturi L, Spitalnik SL, Shakin-Eshleman SH (1998) The amino acid following an Asn-X-Ser/Thr sequon is an important determinant of N-linked core glycosylation efficiency. Biochemistry 37: 6833-6837
26. Novick P, Zerial M (1997) The diversity of Rab proteins in vesicle transport. Curr Opin Cell Biol 9: 496-504
26a.Olson FJ, Johansson MEV, Klinga-Levan K, Bouhours D, Enerbäck L, Hansson GC, Karlsson NG (2002) Blood group A glycosyltransferase occurring as alleles with high sequence difference is transiently induced during a nippostrongylus bransiliensis parasite infection. J Biol Chem 277 (17): 15044-15052
27. Pelham HRB, Rothman JE (2000) The debate about transport in the golgi - two sides of the same coin? Cell 102: 713-719
28. Raghunath M, Kielty CM, Steinmann B (1995) Truncated profibrillin of a Marfan patient is of apparent similar size as fibrillin: intracellular retention leads to over-N-glycosylation. J Mol Biol 248: 901-909
29. Riley RS, Forney JR, Bradley CA, Dunn DD (1990) Alpha-1-antitrypsin: New perspectives and clinical applications. Clin Immunol 10: 95-99
30. Rothman JE, Wieland FT (1996) Protein sorting by transport vesicles. Science 272: 227-234
31. Rucks EA, Fraylick JE, Greene DM, Vincent TS, Olson JC (2002) The role of exoenzyme S in the mechanism of pathogenesis of pseudomonas aeruginosa. FASEB J 16 (4): A203 Abstr. 180.9
32. Rybin V, Ullrich O, Rubino M, Alexandrov K, Simon I, Seabra MC, Goody R, Zerial M (1996) GTPase activity of Rab 5 acts as a timer for endocytic membrane fusion. Nature 383: 266-269
33. Schachter H (1994) Molecular cloning of glycosyltransferase genes. In: Fukuda W, Hindsgaul O (eds.) Molecular Glycobiology. IRL Press at Oxford University
34. Schachter H (2001) The clinical relevance of glycobiology. J Clin Invest 108: 1579-1582
35. Schroeder TH, Lee MM, Yacono PW, Cannon CL, Gerceker AA, Golan DE, Pier GB (2002) CFTR is a pattern recognition molecule that extracts pseudomonas aeruginosa LPS from the outer membrane into epithelial cells and activates NF-κB translocation. PNAS 99 (10): 6907-6912
36. Segev N (2001) Ypt and Rab GTPases: insight into functions through novel interactions. Curr Opin Cell Biol 13: 500-511
36a.Smith RS, Iglewski BH (2003): Pseudomonas aeruginosa quorum-sensing systems and virulence. Curr Opin Microbiol 6: 56-60 36b.Smith RS, Iglewski BH (2003): Pseudomonas aeruginosa quorum sensing as a potential antimicrobial target. J Clin Invest 112 (10): 1460-1465
37. Stenmark H, Parton RG, Steele-Mortimer O, Lütcke A, Gruenberg J, Zerial M (1994) Inhibition of rab 5 GTPase activity stimulates membrane fusion in endocytosis. EMBO J 13 (6): 1287-1296
38. Ujvari A, Aron R, Eisenhaure T, Cheng E, Parag HA, Smicun Y, Halaban R, Hebert DN (2001) Translation rate of human tyrosinase determines its N-linked glycosylation level. J Biol Chem 276: 5924-5931
38a.Vaughan L, Lorier MA, Carrell RW (1982) α1-antitrypsin microheterogeneity. Isolation and physiological significance of isoforms. Biochim Biophys Acta 701: 339-345
39. Wieland FT, Gleason ML, Serafini TA, Rothman JE (1987) The rate of bulk flow from the endoplasmic reticulum to the cell surface. Cell 50: 289-300
39a.Wilmore DW (1977) Impaired gluconeogenesis in extensively injured patients with Gram-negative bacteremia. Amer J Clin Nutr 30: 1355-1356
40. Würtele M, Wolf E, Pederson KJ, Buchwald G, Ahmadian MR, Barbieri JT, Wittinghofer A (2001) How the pseudomonas aeruginosa Exo S toxin downregulates Rac. Nature Structural Biology 8: 23-26
41. Zerial M, McBride H (2001) Rab proteins as membrane organizers. Mol Cell Biol 2: 107-117

## Patentansprüche

1. Verfahren zur Herstellung eines hyperglykosylierten Proteins umfassend das Behandeln eukaryotischer Zellen, die das Protein exprimieren, mit Pseudomonas.

2. Das Verfahren gemäß Anspruch 1, wobei das hyperglykosylierte Protein aus den eukaryotischen Zellen oder aus dem Extrazellulärraum der eukaryotischen Zellen isoliert wird.

3. Das Verfahren gemäß Anspruch 1 oder 2, wobei die eukaryotischen Zellen in Zellkultur mit Pseudomonas behandelt werden.

4. Das Verfahren gemäß einem der Ansprüche 1 bis 3, wobei die eukaryotischen Zellen Säugertierzellen sind.

5. Das Verfahren gemäß Anspruch 4, wobei das hyperglykosylierte Protein in einem nichtmenschlichen Säugetier hergestellt wird und das Säugetier mit Pseudomonas behandelt wird.

6. Das Verfahren gemäß Anspruch 5, wobei das Säugetier mittels Infusion in ein Blutgefäß oder Lymphgefäß mit Pseudomonas behandelt wird.

7. Das Verfahren gemäß Anspruch 5 oder 6, wobei das hyperglykosylierte Protein aus Blutplasma, Lymphe oder Milch des Säugetiers gewonnen wird.

8. Das Verfahren gemäß einem der Ansprüche 1 bis 7, wobei die eukaryotischen Zellen mit einer exprimierbaren DNA transformiert sind, die für das Protein kodiert.

9. Das Verfahren gemäß einem der Ansprüche 1 bis 8, wobei das Protein aus der folgenden Gruppe ausgewählt ist: Blutgerinnungsfaktoren, Polypeptidhormone, Immunproteine, Cytokine, Interferone, α1-Antitrypsin, Wachstumsfaktoren.

10. Hyperglykosyliertes Protein erhältlich gemäß des Verfahrens eines der Ansprüche 1 bis 9.
